# EUROPEAN PATENT SPECIFICATION

(11) **EP 1 996 274 B1**
(45) Date of publication and mention of the grant of the patent: **14.09.2016**
(21) Application number: 07758117.1
(22) Date of filing: 08.03.2007
(51) Int. Cl.: B65D 47/00, A61M 39/10, A61M 39/16, A61M 39/26, A61M 39/20

(54) **ANTI-CONTAMINATION COVER FOR FLUID CONNECTIONS**
ANTIKONTAMINATIONSABDECKUNG FÜR FLUIDVERBINDUNGEN
COUVERCLE ANTI-CONTAMINATION POUR UN RACCORD POUR PASSAGE DE FLUIDE

(30) Priority: 08.03.2006 US 780426 P; 15.02.2007 US 890186 P
(43) Date of publication of application: 03.12.2008
(73) Proprietor: Dotted Intellectual Property LLC, Durham, NC 27703 (US)
(72) Inventor: JARNAGIN, Scott, P., Seattle, WA 98105 (US); PETERS, Bryan, Raleigh, NC 27615 (US); MOSLER, Theodore, J., Raleigh, NC 27614 (US); KOROGI, Todd, M., Raleigh, NC 27614 (US)
(74) Representative: Thum, Bernhard
(86) International application number: PCT/US2007/063534
(87) International publication number: WO 2007/103998

(56) References cited:
- WO-A1-98/48872
- US-A- 5 385 372
- US-A- 5 385 372
- US-A- 5 398 837
- US-A- 5 398 837
- US-A- 5 951 519
- US-A1- 2004 030 321

## Description

### CROSS-REFERENCH TO RELATED APPLICATION

### FIELD

This invention relates to fluid connector covers adaptable and more particularly to passively activated fluid connector covers. The covers may be impregnated or contain an anti-microbial agent to eliminate or reduce contamination of an access site of the fluid connector.

### BACKGROUND

Existing fluid connectors are typically designed without a covering for protecting the access site while it is not in use. Some access sites include screw threads which allow them to more easily be connected to IV tubing during lengthy accesses. However, most external fluid connectors normally remain uncovered and completely exposed with little or no barrier to potential contaminants. Contaminants may include, but are not limited to, germs, bacteria, air, dirt, clothing, skin and perspiration.

Because of the exposure of the fluid connector and access site to contaminants, the use of an aseptic cleansing procedure is typically required prior to accessing the site. Using currently accepted techniques, the cleansing process alone involves several steps and the use of numerous materials for each port access. In order to assure an aseptic environment, a second person may be needed to assist the first with the cleansing process. The clinician may not disinfect the access site for various reasons, including forgetting to do so, not having the appropriate disinfectant swab readily available, being in a hurry, not understanding the importance or a combination of these issues.

When access to the fluid connector is complete, it will again come into contact with the patient's skin or clothing and immediately become susceptible to contamination, requiring repetition of the above procedure the next time the access site is accessed. The cleansing procedure, even if properly followed, is very time consuming. Materials must be gathered prior to access, and an assistant may be required before the procedure can be started. The more frequent the access, the greater the risk of subsequent contamination.

Recent scientific studies suggest that the external access site of a fluid connector may be the place of origin of bacteria infecting the device. These studies recommend that manipulation of the access site be kept at a minimum, and that a more rigorous approach to aseptic technique be undertaken such as the time consuming, expensive and difficult measures as discuss above. While studies have suggested that the hub of catheter devices be properly covered, such studies fail to propose any cover design apart from indicating covers are needed to protect against contamination.

While external injection port covers directed to maintaining sterility of catheter hubs have been disclosed, these external injection port cover designs require that the cover include threaded elements to securably cover the corresponding threaded elements of the fluid connector. Thus, the cover requires manual manipulation for removal and replacement thereof on the fluid connector and may require a unique design each fluid connector. An example of a connector with an integrated cover is described in US 5,385,372. An example of a flask top that can be actuated with one digit is described in US, 5,398,837. Minimization such manipulation and universal design of an aseptic cover for fluid connectors would improve the compliance and sterility of the fluid connector manipulations.

### SUMMARY

To address the aforementioned problems, a fluid connector having a cover for fluid connectors is herein described, which may be adaptable to swappable, luer-activated needle-free valves of various forms e.g. luer locking adapters, y-sites, vial adapters, as well as pre-slit septums accessible by blunt cannula, and septums accessible by needles. The cover may passively cover the access site of fluid connectors of various shapes and types. The cover may provide anticontamination material to the access site. After access, the cover may passively re-position over the access site to provide a clean and aseptic condition at the access site.

A fluid connector according to claim 1 is provided. Preferable embodiments are set-forth in the dependent claims.
and a cover member. The cover member is integral with the distal end of the rigid arm and positioned to cover the access site. The cover member is displaceable from an initial position covering access site to a second position uncovering the access site upon displacement of the rigid arm.

A cover adapter is provided comprising a body member securable to a fluid connector having an access site, a rigid arm with a proximal and distal end, a flexible member joining the rigid arm to the body member between the proximal and the distal end of the rigid arm, and a cover member. The cover member is integral with the distal end of the rigid arm and positioned to cover the access site of the connector. The cover member is displaceable from an initial position covering the access site to a second position uncovering the access site upon displacement of the proximal end of the rigid arm.

A fluid connector is provided comprising a housing. The housing comprises at least one fluid access site and attachment members, a body member secured to the housing with corresponding attachment members, and a flexible member extending from the body member and at least partially surrounding the housing with at least a portion of the flexible member secured to the housing. A rigid arm having a proximal end and a distal end is joined to the body member between the proximal end and the distal end. A cover member is secured to the distal end of the rigid arm and covers the access site.

A housing for a fluid connector cover is provided. The housing comprises an access site, attachment members positioned on the housing, and at least one housing retaining member projecting from the housing. The attachment members and the at least one housing retaining member cooperatively receive a fluid connector cover adapter. The adapter comprises a body member having corresponding attachment members securable to the fluid connector attachment members, a flexible member coupled to the body member and securable by the at least one housing retaining member, and a rigid arm having a proximal end and a distal end. The rigid arm is joined to the body member between the proximal end and the distal end. A cover member, secured to the distal end of the rigid arm, is positioned to cover the access site.

A fluid connector comprising a housing is provided. The housing comprises at least one fluid access site, attachment members, a body member secured to the

### BRIEF DESCRIPTION OF THE FIGURES

The following FIGs 11 to 16 show embodiments according to the invention, while the remaining FIGs 1 to 10 and 17 to 33B show examples which are useful for understanding the invention.
FIG. 1 is a perspective view of a fluid connector cover.
FIG. 2A is a perspective view of a fluid connector cover.
FIG. 2B is a perspective view of the fluid connector of FIG 2A with external device engaged.
FIG. 3 is a perspective view of a fluid connector cover on a y-body connector.
FIG. 4 is a perspective view of a fluid connector cover.
FIG. 5 is a perspective view of a fluid connector cover.
FIG. 6 is a perspective view of a fluid connector cover.
FIG. 7 is a side cross-section view of the cover portion of the fluid connector of FIG. 6.
FIG. 8 is a perspective view of a fluid connector cover.
FIG. 9 is a perspective view of a fluid connector cover.
FIG. 10 is a perspective view of a cover adapter.
FIG. 11 is a perspective view of a fluid connector cover.
FIG. 12 is a perspective view of the fluid connector cover of FIG. 11 in an activated state.
FIG. 13 is a perspective view of the fluid connector cover on a y-body connector.
FIG. 14 is a perspective view of the fluid adapter of FIG. 13 in an activated state.
FIG. 15 is a perspective view of a cover adapter.
FIG. 16 is a perspective view of a fluid connector housing.
FIG. 17 is a perspective view of a fluid connector cover.
FIG. 18 is a perspective view of the fluid connector cover of FIG. 17 in an activated state.
FIG. 19 is a perspective view of a cover adapter.
FIG. 20 is a perspective view of a fluid connector cover.
FIG. 21 is a perspective view of a fluid connector cover.
FIG. 22 is a perspective view of the fluid connector cover of FIG. 21 prior to engaging an external device.
FIG. 23 is a perspective view of a fluid connector cover.
FIG. 24 is a perspective view of a cover adapter.
FIG. 25 is a perspective view of the fluid connector cover of FIG. 23 in an activated state.
FIGs. 26A and 26B are cross-sectional and perspective views, respectively, of a cover adapter.
FIG. 27 is a cross-sectional view of a cover adapter.
FIG. 28 is a perspective view of a cover adapter.
FIG. 29 is a detail side view of a cover adapter.
FIG. 30 is a perspective view of a cover adapter.
FIG. 31 is a perspective view of a fluid connector cover.
FIG. 32 is a perspective view of the fluid connector cover of FIG. 31 with an engaged external device.
FIGs. 33A and 33B are perspective views of fluid connector covers with a vial adapter in an initial and activated state, respectively.

### DETAILED DESCRIPTION

A fluid connector cover for fluid connectors is herein described, which may be adaptable to swabable, luer-activated needle-free valves of various forms e.g. luer locking adapters, y-sites, vial adapters, penetetrable septums accessible by blunt cannulaor by needles. The cover may passively cover the access site of a fluid connector such that a user must intervene to expose the access site. The cover may provide anti-contamination material to the access site. This may be accomplished by several features of the cover, as described herein and summarized in the figure descriptions that follow.

As used herein, the term "access site" refers generally to a fluid port providing entry by a piercing, penetrating, or inserting device. By way of example, an access site includes the female port of a needle-free valve and a penetetrable septum accessible by cannula e.g., blunt cannula, respectively. Penetrable septum may include pre-slit and un-slit septum, The access sit includes the adjacent area in proximity to the access site.

The cover may at least partially cover the access site of the connector. The cover may provide a covering relationship with the access site. The covering relationship includes direct contact or being in close proximity to the access site. This may prevent many of the infections typically caused by touch contamination, for example, at the injection site of infusion devices.

The fluid connector cover may be of a passive design, which generally refers to a requirement that a user actively forces the cover off of the access site, thus, the fluid connector cover will be positioned at or over the face of the access site in its natural state. This feature may guard against misuse and provide for the face of the valve remaining protected from possible contamination. For example, using the fluid connector cover herein described, nurses, clinicians or other medical professionals may not need to re-cover the fluid connector. Therefore, the possibility of the cover falling off or being lost and/or discarded and exposing the access site is reduced or eliminated.

The fluid connector cover herein described may be designed such that a minimal number of components are added to an existing fluid connector. These designs and other features of the fluid connector cover herein described may utilize many of the parts already found in fluid connectors such as needle-free valves. This may limit additional parts and associated costs as well as new or complicated assembly procedures. In addition, changes to current needle-free valve parts may be done without complicating tooling, and many of the herein disclosed designs may be made with an open/close mold action, minimizing production cost.

The fluid connector cover herein described may be integrated with connectors comprising access sites. The fluid connector cover herein described may be integrated into the connector housing, by way of example, through a flexible beam connection, strap connection, or snap means. Other ways of integrating the fluid connector cover to the connector housing may include a cylindrical clip adapted to secure around at least a portion of the housing of the connector, or a strap fitted around the housing. The fluid connector cover herein described may provide for a mating hole and lug configuration such that activation of the cover provides a pivoting action. The fluid connector cover herein described may be adapted to connectors comprising integrated standard threads to provide connection with a female luer, and/or snap on or rest over an access site of a fluid connector. The fluid connector cover herein described may comprise cutouts such as to allow snap-fit-like flexibility for variable sized valves.

The fluid connector cover herein described may stay external to a fluid connector housing and may touch the surface of the access site. The cover may require removal before accessing the access site and to actuate the fluid pathway.

The term "fluid" as used herein, refers to gas, liquid or a combination of gas and liquid.

The fluid connector cover may function "passively". Functioning "passively" means that unless a practitioner actively forces the cover off of the access site, the fluid connector cover remains in a covering relationship with the access site, at least until and before the first use of the connector. By way of example, the natural state of the fluid connector cover is covering the access site. The passive fluid connector cover may provide against misuse in the field and may ensure the exposed surface of the access site remains covered and/or protected. The passive fluid connector cover may eliminate or reduce the need for nurses, clinicians or other medical professionals to re-cover the valve and therefore reduce or eliminate the possibility of the cover falling off on its own and/or exposing the access site to contamination. The passive functionality includes, for example, a pre-loaded returning force allowing the cover to return to the access site upon disconnect. The returning force may include a preload condition e.g., a load where the access site will remain covered in the event that the returning force yields under normal operation. By way of example, the returning force may be supplied with a spring, elastomeric material or cantilevered beam. The pre-load force may create a condition where the access site will remain covered should the pre-load elements yield under normal operation.

The fluid connector cover may operate substantially automatically upon engagement by an external device with the access site without user intervention of the fluid connector cover. For example, the cover member may comprise extending flexible arms which provide for deflection of the cover member when the external device engages the access site. The cover member may be comprised of two or more mating sections which form a cover over the access site when in an initial state. The mating section may be deflected away during engagement. Flexible arms may provide a load to the mating sections in the initial state such that upon disengagement of the external device the segments may return to the initial state covering the access site.

A fluid connector cover may comprise a housing. The housing and other components attached or adapted for attachment thereto may be of plastic construction or may be fabricated out of one or more materials designed to withstand chemical attack from substances, such as solvents and IV drugs. Materials include for example, thermoplastics, engineering thermoplastics, filled or unfilled, and composites. Thermoplastics include materials such as polybutylene terephthalate PBT, cyclic olefinic copolymers COC's , thermoplastic polyurethanes TPU, rigid polyvinyl chloride PVC, and polycarbonate PC.

The fluid connector cover may comprise a body member. The body member may be integral with the connector housing. The body member may provide attachment means for securably attaching to the connector housing.

The fluid connector cover comprises a rigid member. The rigid member may be integral with the connector housing or may be integral with the body member of the connector cover. The rigid member may be cooperatively coupled with a cover member which provides for covering the access site.

The fluid connector cover may comprise a flexible member. The flexible member may join the rigid member to the body member or may join the rigid member to the connector housing. The flexible member may comprise a thinned wall or narrowed width section of the rigid member. The flexible member may provide a load to the rigid member.

The use of the phrases "flexible member" and "rigid member" are to be taken in context as elements used together in the operation of the fluid connector cover and not necessarily a representation of a material property of the members. For example, rigidity and flexibility of the members may be provided by varying thickness and cross-sectional area of a single material, or the rigidity and flexibility of the members may be provided by using a thermoplastic material and an elastomeric material.

The fluid connector cover herein described may include a shroud element. The shroud element at least partially surrounds the cover member such as to cover the sides of the access site. A portion of the shroud element may provide for accepting of the access site upon return of the cover member to its initial state. The portion of the shroud may include a cut out or missing section, for example, positioned opposite the rigid member attachment point to cover member.

Any surface or combination of surfaces of fluid connector cover herein described may include and/or be impregnated, and/or otherwise covered, with an aseptically effective material. The aseptically effective material may comprise an anti-microbial agent. The cover member may comprise separate and/or replaceable element containing an anti-microbial agent. The separate element may be adapted to absorb new or additional anti-microbial agents. For example, the separate element may include a sponge, foam or absorbable material sized to be received by the cover member. The separate element may be secured temporarily or permanently to the cover member. The separate element may be secured by one or more securing elements integral with the cover member or shroud.

The fluid connector cover herein described may integrate a cover member with a body member or fluid connector housing by way of a flexible member. The flexible member may be shaped as a beam or arm. The cover member may be positioned at a distal end of the arm, the arm being connected to the flexible beam connected to the body or the housing, for example, in a lever arm-fulcrum flexible beam relationship. Pressing the proximal portion of the arm activates the device by flexing the beam, which moves the cover away from the access site in an arc-like motion.

The fluid connector cover herein described may comprise a cover adapter having attachment means for securely fastening with the fluid connector housing. For example, the cover body may comprise a ball/pin and socket, interference-type snap-on structure, a clip or combinations thereof. The fluid connector cover herein described may comprise a clip member formed of the body member for surrounding at least a portion of the connector housing. The clip member may provide for securing the cover adapter onto the connector housing and may further utilize joining methods, such as adhesives or ultrasonic welding, to prevent relative motion between the connector housing and the cover adapter. The cover adapter may further comprise a flexible beam and arm as described above, such that pressing the lower portion of the arm moves the cover away from the access site of the connector housing in an arc-like motion.

The fluid connector cover herein described may comprise a threaded cover member with an integrated strap, the strap having at its opposite end the cover member. The strap may be attached to the connector housing such that the strap is free to rotate around the connector housing body. The rotation of the strap about the connector housing may provide for facile threaded engagement or disengagement of the cover member. An alternate threaded cover member to that previously described. Thus, the strap may be integrated with the connector housing while the cover member, located at the opposite end of the strap, is free to rotate relative to the strap.

The fluid connector cover herein described may be integrated with a fluid connector housing such that the cover member may be vertically translatable and arcuately rotatable about the connector housing. In this configuration, the cover may be pushed down over the access site and/or any threaded elements. Thus, the cover member may comprise integral projecting flexible arms, the arms comprising tracks which securably attach to the housing to provide vertical and arcuate motion to the cover member relative to the connector housing. The cover member may be of a cup-like shape. The cover member may be adapted to snap-fit on the access site, for example to keep the underside covering surface of the cover member flush against the face of the access site. The cover member may also utilize the thread forms on the access site as the mating snap elements.

The fluid connector cover herein described may comprise one or more flexible arms proximally integral with a fluid connector housing, or the flexible arms may be adapted to securably attach thereto. The flexible arm may be cooperatively coupled with the cover member at the distal end of the flexible arm such that applied force upon the flexible arm toward the connector housing translates the cover member from the access site to provide access thereto.

The fluid connector cover may further comprise a flexible member that surrounds the housing and may be cooperatively coupled to the fluid connector cover. The flexible member may provide a restoring force to the fluid connector cover such that the fluid connector cover returns to its initial state covering the access site. The flexible member may be combined with the previously described fluid connectors. Thus, for the fluid connector cover comprising rigid arm and beam elements, the flexible member may be used to provide a restoring force for or load to the rigid arm.

For example, applied force, such as digital pressure from a user, to the lower portion of the rigid arm provides rotation about a clip-like means and, in turn, the beam flexes and creates the restoring force returning the cover over the access site. The connector housing may be a connection point for the clip-like means, and may also restrict the movement of the beam, causing the part of the beam to flex and/or creating or complementing the restoring force to re-cover the access site. The flexible arms may snap-on to corresponding lugs integral with the fluid connector housing. The lugs may be designed such that the flexible arms deflect when the clip is activated. The flexible arm may be, for example, of a U-, V-, or W-shape.

The fluid connector cover may comprise attachment means. Attachment means may include a rotatable clip-like means to provide for rotation of the fluid connector cover body around the outside surface of the connector housing. The cover body may include a flexible segment joining a rigid arm to the cover body, the rigid arm distally terminating in a cover member. The connector housing may comprise a connection point for the clip-like means and a ramp element cooperatively engage the rotatable clip-like means to provide deflection of the flexible segment. The flexible segment may provide a load when the clip-like means are rotated and cooperatively engaged with the ramp element. Deflection of the flexible segment moves the cover member from the access site. The load may re-position the cover member after the clip-like means are disengaged with the ramp element.

The fluid connector cover may comprise an elastomeric material. The elastomeric material may secure the cover member over the access site and/or provide a returning force. The elastomeric material may be combined with the previously disclosed fluid connectors. The elastomeric material may be integrated with the clip-like means or other attachment members. The elastomeric material may be integrated, for example, over-molding or adhesive bonding. Attachment means connecting the fluid connector cover body to the connector housing access site may be a pivoting connection. The lower portion of the attachment means may be covered with the elastomeric material to improve tactile feel, finger control, and/or ergonomic stability. The attachment means may be made entirely of an elastomeric material such that the rigid member and flexible member functioning may be controlled by material thicknesses and cross-sections. The elastomeric material may provide for improved sealing between the cover member and the access site by reducing or eliminating any gaps between the access site and cover member. The elastomeric material may provide for conformal sealing between the cover member and a non-planar access site, for example. Various elastomeric materials may be used, such as, but not limited to, reaction-injection molded RIM elastomers, silicones, polyurethanes, thermoplastic elastomers TPE e.g., SANTOPRENE™, and synthetic polyisoprenes.

Where applicable, connection geometry for attachment members that do not integrate with the connector housing may be optimized for manufacturability of the connector. For example, an injection-molded connector housing may comprise attachment members positioned such that the mold tool only requires simple modification rather than retooling. As a means of cost reduction, embodiments of this concept may be integrated into the intended access site. The fluid connector cover may comprise a cover member connected to a distal end of a rigid member, the rigid member joined to the connector by a flexible member arm. Applied force to the rigid member provides flex which moves the cover away from the access site in an arc-like motion.

The fluid connector cover may be integrated with a connector housing for example, using a flexible beam connection, strap connection, or snap means. Other ways of integrating are through a cylindrical clip that fits around the body of the housing or a strap fitted around the housing. Standard threads may be integrated into the fluid connector cover to provide a securable connection with a female luer, or may snap-on or rest over the access site. For example, cutouts in the fluid connector cover body may allow sufficient flexibility for snap-fitting onto any size fluid connector housing.

The cover member may be integrated with the connector housing or may be a separate component of the fluid connector cover. The cover member may be cooperatively joined or integral with a rigid or flexible member, for example, having the shape of an arm connected to the connector housing.

The cover may be adaptable to commercially available connectors including, but not limited to, Cardinal Health SmartSite® and SmartSite® Plus, ICU Medical Clave®, CLC2000®, and TEGO™, BD Posiflow™ and Q-Syte™, Baxter FLOLINK, CLEARLINK, and INTERLINK, B|Braun ULTRASITE®, Medegen MaxPlus™, Halkey-Roberts Swabable Luer Valve, RyMed Technologies InVision-Plus®, Vygon Bionector®, and Borla bSite™. Additionally, the cover may be adaptable to female connectors of stopcocks and IV manifolds.

Methods of preventing or eliminating contamination of an access site of a fluid connector are provided. As used herein, the term "prevent" and its grammatical equivalents refer to any reduction of the level and/or virulence contamination of the access site. As used herein, the term "contamination," and its grammatical equivalents refer to material not intended to be present on or in the access site. Preventing or eliminating contamination of an access site of a fluid connector may include, for example, providing a passive cover member positioned in a covering relationship with the access site. The cover member may prevent or eliminate contact of the access site with the user, material and/or surfaces that may contain virulent material. The cover member may passively cover the access site until user intervention provides access to the access site, for example, using digital pressure applied to the fluid connector cover.

The fluid connector herein described may contain an aseptically effective agent to provide infection controlling properties to the connector. Medical articles are frequently fabricated from polymeric materials such as polypropylene, silicone rubber, ABS, or polyurethane by molding and extruding techniques. Imparting aseptically effective properties to such medical devices made from polymeric materials includes, for example, incorporating an anti-microbial agent into the material during the process of forming the device.

As used herein, the term "aseptically effective agent" refers to chemicals or compositions of matter that either kill or prevent growth of virulent organisms. A preferred aseptically effective agent is an anti-microbial agent. Anti-microbial agents include antibacterial agents which kill bacteria, antiviral agents which kill viruses, antifungal agents which kill fungi, and anti-parasitic drugs which kill parasites. Aseptically effective agents include biocides and antibiotics.

As used herein, the phrase "microorganisms," refers to, for example, Gram positive and Gram negative bacteria, including resistant strains thereof, for example methicillan-resistant Staphylococcus aureus MRSA, vancomycin-resistant Enterococci VRE and penicillin-resistant Streptococcus pneumoniae PRSP strains. Preferably, it is defined to include all bacteria Gram+, Gram- and acid fast strains and yeasts such as Candida albicans. Most preferably, it is defined to include all bacteria Gram+, Gram-, and acid fast, yeasts, and both envelope and naked viruses such as human influenza, rhinovirus, poliovirus, adenovirus, hepatitis, HIV, herpes simplex, severe acute respiratory syndrome SARS, H5N1 viruses, and avian flu.

The term "biocides" refers generally to a chemical agent which inactivates living microorganisms. Biocides include, for example, agents of broad spectrum activity, agents that inhibit growth e.g., bacteriostatic, fungistatic, or sporistatic and agents that kill the target organism e.g., bactericidal, fungicidal, sporicidal, or virucidal.

As used herein, the term "containing," when referenced in regard to an aseptically effective agent, refers to any method of incorporating the agent to a fluid connector cover. This may include, for example, melt addition of the agent to a polymer melt during extrusion or manufacturing; topical application methods; absorbable impregnated pads; and other non-standard methods such as plasma treatment, electrostatic attachment, vapor deposition, radiation surface graft copolymerizations using for example UV, gamma rays and electron-beam radiation sources, or the use of chemical initiation to produce graft copolymerized surfaces having anti-microbial activity, etc.

The aseptically effective agent containing fluid connector cover preferably rapidly inhibits and controls the growth of microorganisms. For example, an anti-microbial agent may provide a reduction in the concentration of a broad spectrum of microorganisms by a magnitude of at least 1 log₁₀ as measured by shaker flask method, liquid droplet challenge test, and/or aerosol challenge test within about 30 minutes. The anti-microbial agent containing fluid connector cover may lead to a reduction in microbial concentration by a factor of 3 log₁₀ i.e., reduction by 10³ colony forming units per gram of material cfu/g within about 30 minutes. The anti-microbial agent containing fluid connector cover may lead to a reduction in microbial concentration by a factor of 4 log₁₀ or more within about 30 minutes.

The anti-microbial agent containing fluid connector cover may provide at least a 1 log₁₀ reduction in the transfer of a broad spectrum of viable microorganisms when contacting another surface as compared to an untreated control item as measured by the contact transfer protocol, for example, as outlined in U.S. Patent Application Publication No. 2004/0151919, incorporated herein by reference.

The anti-microbial agent containing fluid connector cover may be non-leaching. "Non-leaching" anti-microbial surface is one that passes ASTM E2149-01 testing protocol entitled "Standard Test Method for Determining the Anti-microbial Activity of Immobilized Anti-microbial Agents Under Dynamic Contact Condition." The lack of a zone of inhibition with the treatment agents chosen may demonstrate the active species does not leach from the treated substrate.

Aseptically effective agents may be compounded in thermoplastic resins or elastomers to produce a concentrate which is then dry blended with the virgin resin and co-molded or overmolded. For example, an anti-microbial agent may be generally distributed throughout the bulk of the cover such that enough of the anti-microbial agent is present on or near the access site surface to provide anti-microbial activity. Concentration of the anti-microbial present on or near the surface of the access site may depend on several factors including anti-microbial concentration in the melt relative to the main body of resin or type of resin, processing conditions and thermal history, crystallinity of the resin, and relative thermodynamic compatibility of the resin and the anti-microbial. The anti-microbial may be compatible with thermoplastic resin in the melt for proccesability, and yet may be less compatible with the resin at ambient conditions so that the anti-microbial agent may migrate to a certain extent to the surface of the cover. Processing aids may be used to assist migration of the anti-microbial agent to the surface of the cover in contact with the access site.

Anti-microbial agents that may be used in the fluid connector cover as herein described to provide anti-microbial action, include, but are not limited to, 1-alkyl-1, 5-diazapentane, alexidine, alkyl-amino-alkyl glycine, alkylsulfosuccinate, anatase TiO₂, bamboo extract, benzalkonium chloride, bromo-compound, cetrimide, cetylpyridium chloride, chitosan, chlorhexidine biguanide, chlorhexidine diacetate, chlorhexidine digluconate, citric acid, cyanobutane, dialkyl-dimethyl-phosphonium salt, dithiocarbamate, hexachlorophene, hydrogen peroxide, hydrotropes strong emulsifiers and chao tropic agents alkyl polyglycosides, isothiazolin, neem oil, polyhexamethylene biguanide, polyphenols from green or black tea extract, polyquaternary amine; metal-containing species and oxides thereof, quaternary ammonium compound, quaternary ammonium compounds benzalkonium chloride, quaternary ammonium siloxane, quaternized cellulose and other quaternized polymers, silver salts such as silver sulfadiazine either in particle form or incorporated into support matrix or polymer, stabilized oxidants such as chlorine dioxide, stabilized peroxide urea peroxide, mannitol peroxide, sulfites sodium metabisulfite, thiazole, thiocynate, thione, tourmaline, light-activated porphyrins, triclocarban, triclosan, and salts thereof. Depending on the particular material used and the method of incorporation of the anti-microbial agent into the product, many of the above agents may be used alone or synergistically to achieve product properties of interest.

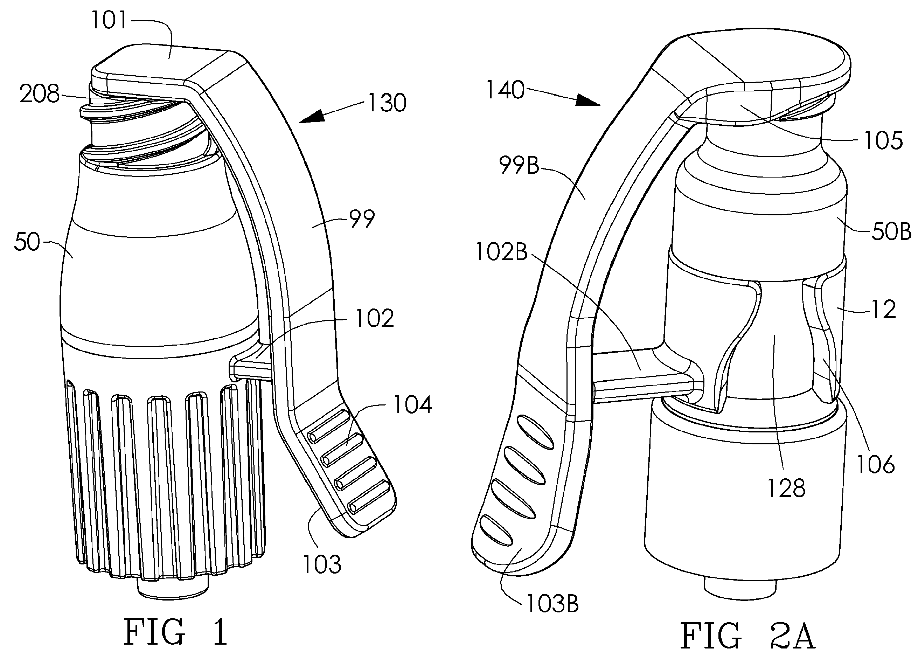

Referring now to the drawings, various illustrative fluid connectors will be described. Referring now to FIG. 1, fluid connector cover 130 may be integrated into connector housing 50 comprising an access site 208. When lower portion 103 of the rigid arm 99 is pushed towards the housing, the upper portion of the arm comprising cover member 101 pulls away from the access site of the housing in an arc-like motion, allowing for insertion of a mating connector. Cantilevered flexible member 102 bends to provide a load or force to substantially return the upper portion of the arm and cover member 101 back over the access site, for example, upon disconnection of the mating connection. Finger grips 104 may be added for finger stability and ergonomic control.

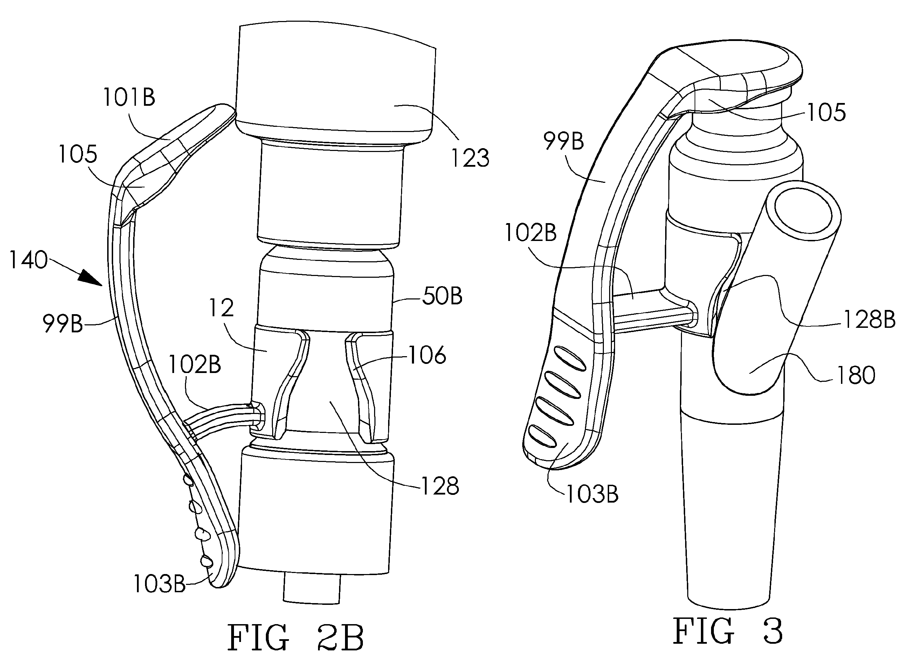

Referring now to FIGs. 2A and 3, fluid connector cover 140 comprises body member 12 non-integrated with connector housing 50b. The body attachment members 106 provide for clip-on and/or adhesion onto different shaped connector housing bodies. Cut-out 128 in the body member may provide attachment on connectors with external geometry protruding from the intended access site body, such as Y-shaped valve 180, as shown in FIG. 3. Shroud 105 may be provided for additional coverage and stability of the cover member. The shroud may extend past the access site.

Referring now to FIG. 2B, a mating connector 123 attached to fluid connector housing 50B with fluid connector cover of FIG. 2A. The flexible member 102B may bend to provide a load as digital force is applied to the lower portion 103B of rigid member 99B. Upon disconnection of the mating connection body, the load in the rigid member may return cover member 101B to the access site.

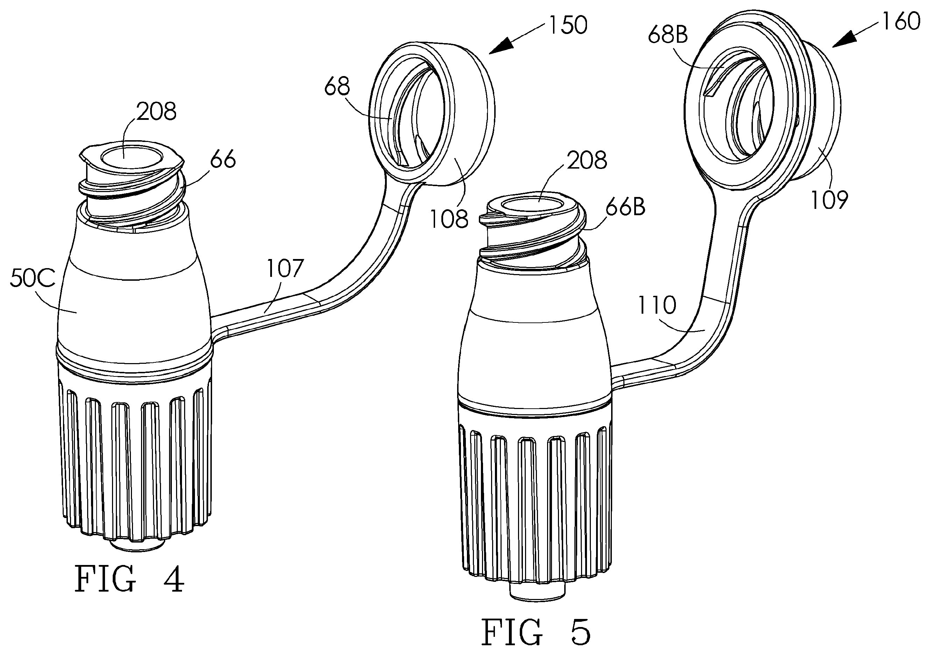

Referring now to FIGs. 4-5, fluid connector cover 150 comprises cover member 108 with threaded element 68, the cover member integrated with a strap 107 having annular clearance around connector housing 50C. Cover member 108 may be screwed onto the threaded element 66 of the housing to cover access site 208 while the strap 107 may rotate around the housing. Fluid connector cover 160 comprises threaded cover member 109 which may be fitted with annular clearance to flexible strap 110 integrated into the housing of the connector. Cover member 109 may turn or spin freely in the strap, for example, while screwing onto threaded elements 66B of connector body.

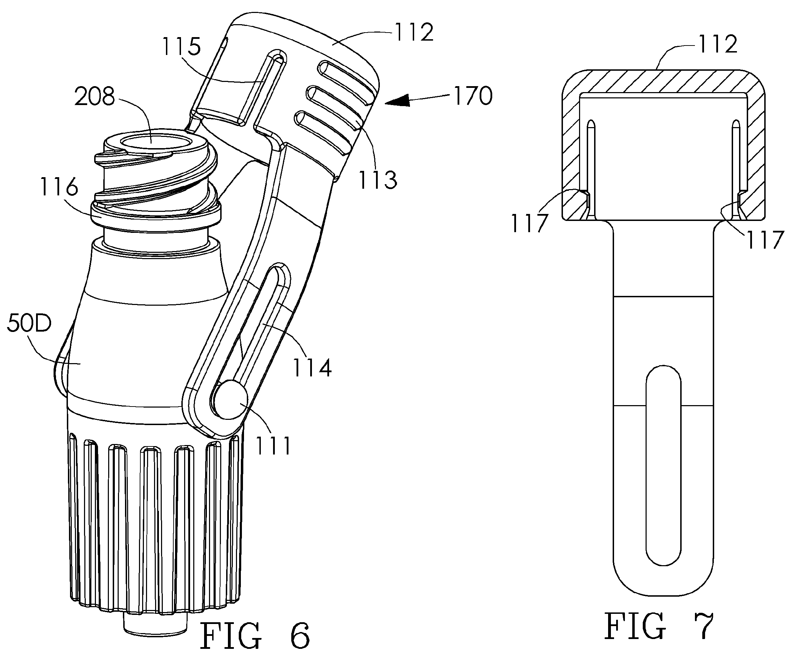

Referring now to FIGs. 6-7, fluid connector member 170 comprise cover member 112 which provides for snap-on functionality of cover member 112 onto corresponding receiving elements or set of elements 116 of connector housing 50D. Cutouts 115 may be provided to allow the cover member walls to flex when engaging elements 116. Groove 114 of the cover member securably attaches onto corresponding attachment members 111 of connector housing 50D. The grooves may allow the cover member to be lifted and swung away from access site 208 in an arc-like motion and may provide a load to return cover member to a position over access site. Finger grips 113 may be integrated into cover member 112 to provide for application and removal of the cover member. Undercuts 117 on cover member 112 may be provided to secure to corresponding engaging elements 116 on housing.

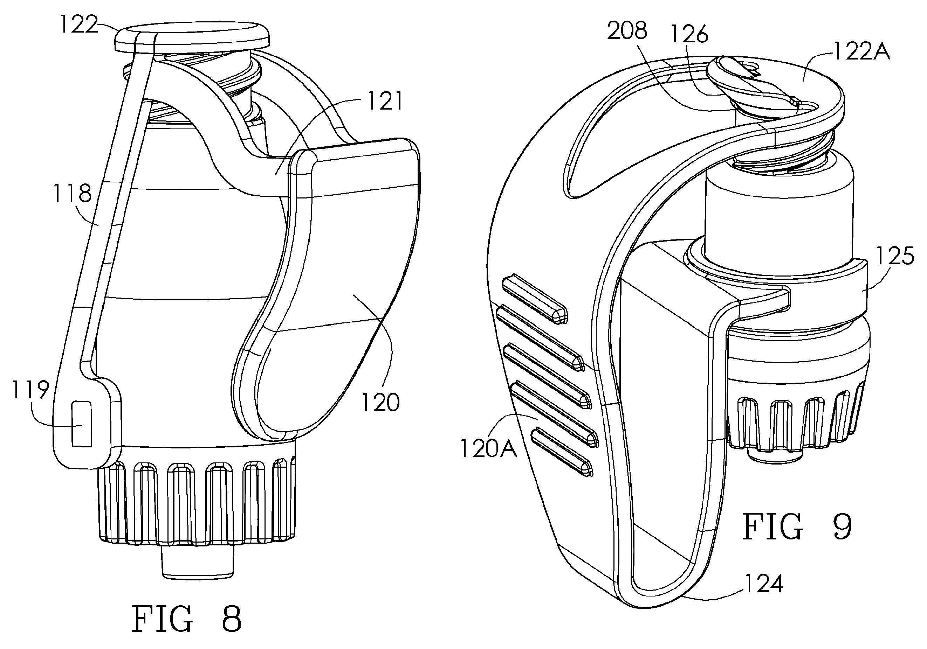

FIGs. 8 and 9 generally depict fluid connector covers where the translation of the cover member corresponds to the direction of the applied force.. Referring now to FIG. 8, actuator 120 may be joined to rigid arm members 121 and flexible arm members 118. Arm members 118 may be secured to housing with snap-on attachment means 119. Arm members 121 may transmit load to flexible linking arms 118 when pressed inward, moving cover member 122 away from the access site. Upon disconnecting, tension in the flexible arm 118 may provide force for the cover member 122 to return back and cover the access site. FIG. 9 actuator 120A may comprise a continuous element 124 having attachment means such as clip-like element 125 securing actuator 120A to connector housing, terminating at cover member 122A. Continuous element 124 comprises opening for providing access to access site 208. Continuous element 124 may be U-, V, or W-shaped such as to provide a load to passively translate cover member 122A back to initial state covering access site. Cover member 122A may include an angled lead-in element 126 to aid in seating cover member 122A upon returning to its initial state.

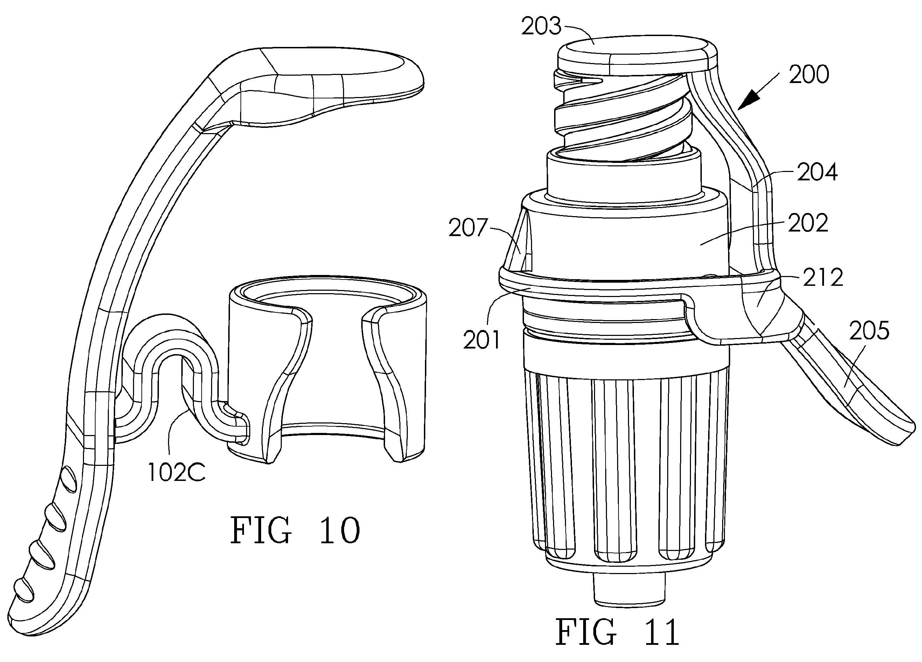

Referring now to FIG. 10, flexible member 102C is shown with alternate beam geometry. The "s" shaped geometry may minimize stress and/or reduce the actuation force. Other shapes and configurations are envisaged such as to provide a force for passively returning the cover member to the access site.

Referring now to FIGs. 11-12, fluid connector cover 200 is shown. Cover body 212 comprising flexible member 201 at least partially surrounds connector housing 202. Rigid arm 204 attaches to cover body between cover member 203 and lower portion 205. Digital force to the lower portion 205 translates cover member 203 off access site 208 in an arc-like motion to expose the access site. Housing retaining means 207 on housing 202 restricts the movement of flexible member 201 providing flexation and load for returning cover member to the access site.

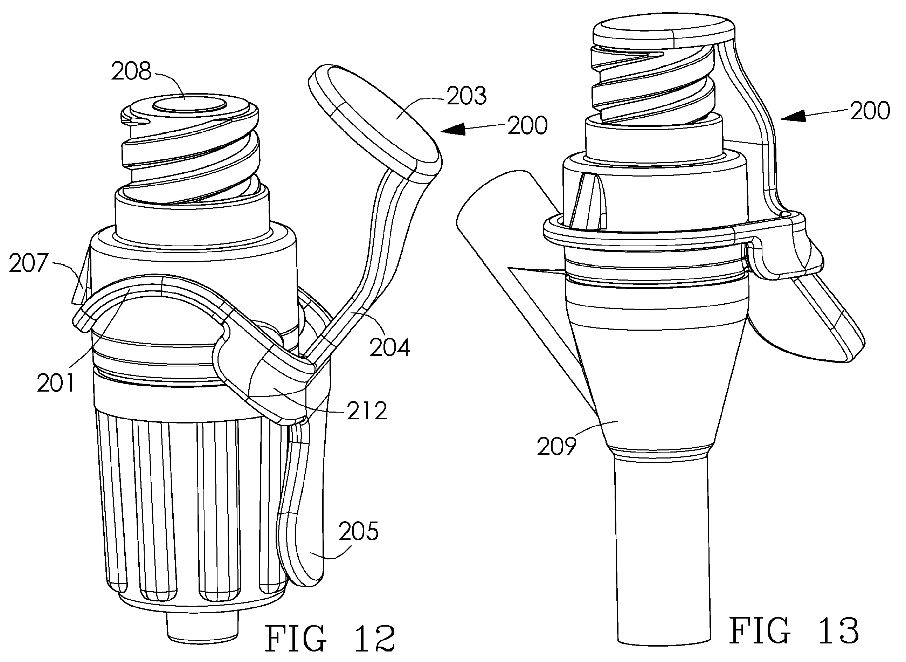

Referring now to FIGs. 13-14, fluid connector cover 200 is shown attached to a y-configuration connector housing 209 in an initial state and in a user-activated state exposing access site 208.

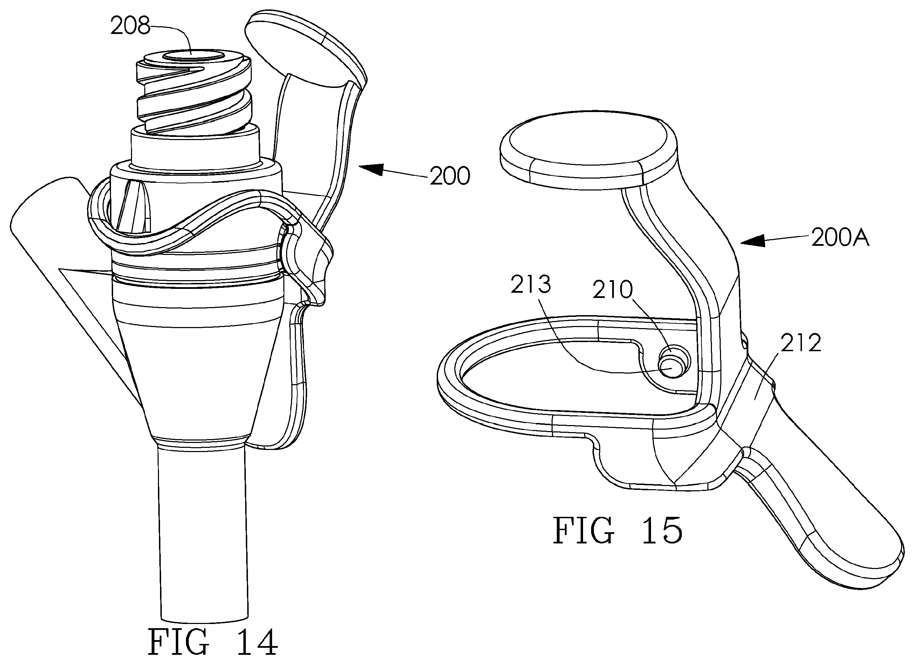

Referring now to FIG. 15, cover adapter 200A of FIGs. 11-14 is shown. Attachment elements 210 may create a pivot point for the cover body 212. Lead-in chamfers 213 may be provided to aid in vertical z-axis assembly of cover adapter to housing.

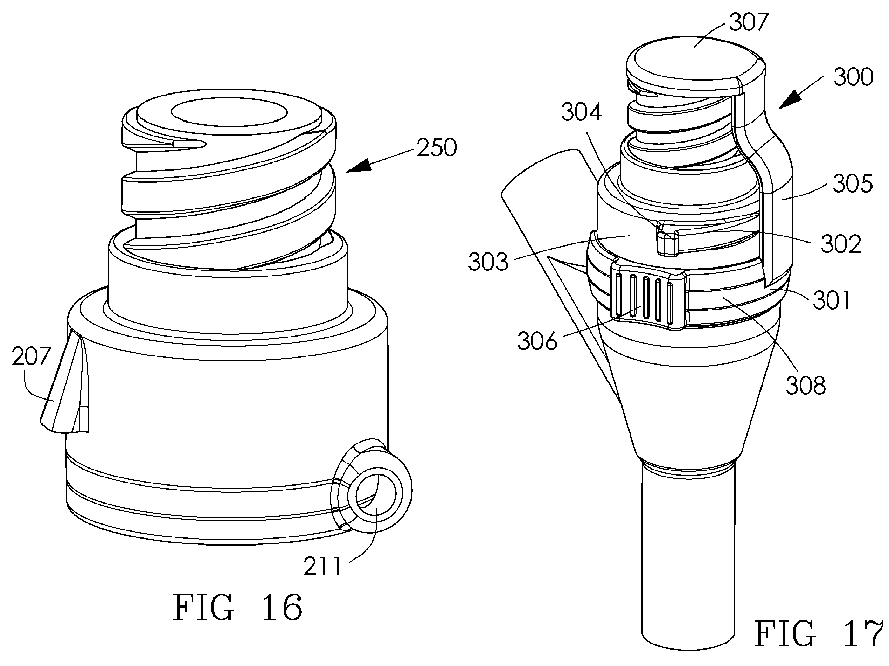

Referring now to FIG. 16, connector housing 250 with corresponding attachment members 211 to attachment elements 210 of FIG. 15 are shown. Attachment members 211 may provide snap-in-place and pivoting of cover adapter 200A.

Referring now to FIGs. 17-18, fluid connector cover 300 is shown in an initial state and an activated state, respectively. Collar 308 connects cover adapter 301 to connector housing 303. Beam-like flexible member 305 connects collar 308 to cover member 307. Rotation about connector housing 303 forces flexible member 305 up ramp 302. Ramp 302 may be integrated on connector housing 303. Finger grips 306 may be added to the collar for manipulation. Rotation of flexible member may be limited by housing retaining member 304. The ramp provides for flexible member 305 and cover member 307 to expose access site 208.

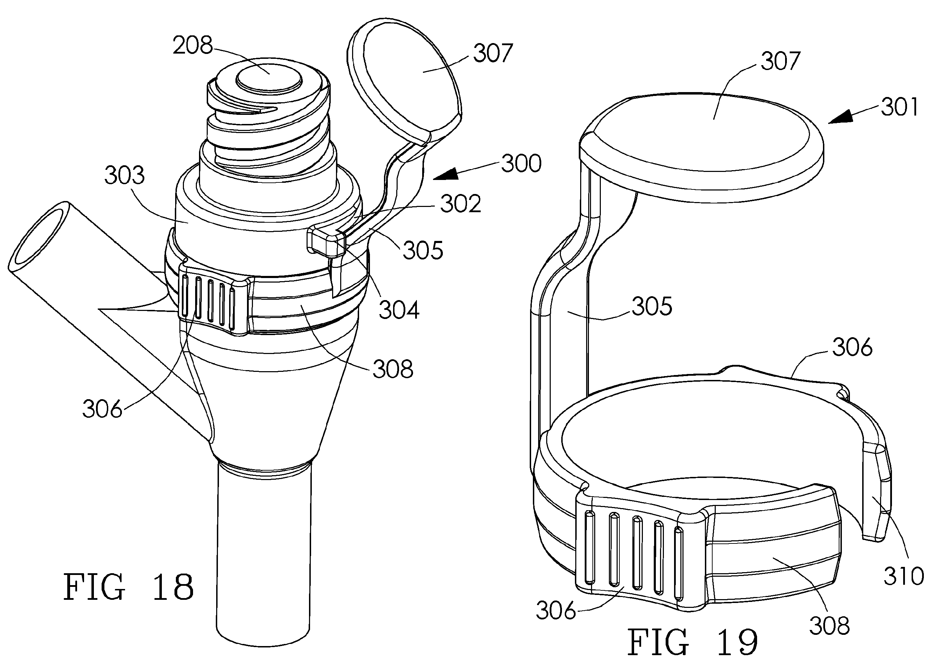

Referring now to FIG. 19, cover adapter 301 of the fluid connector cover of FIG. 18 without connector housing is shown. Cutout 310 of collar 308 provides for snap-on assembly of cover adapter to a fluid connector housing.

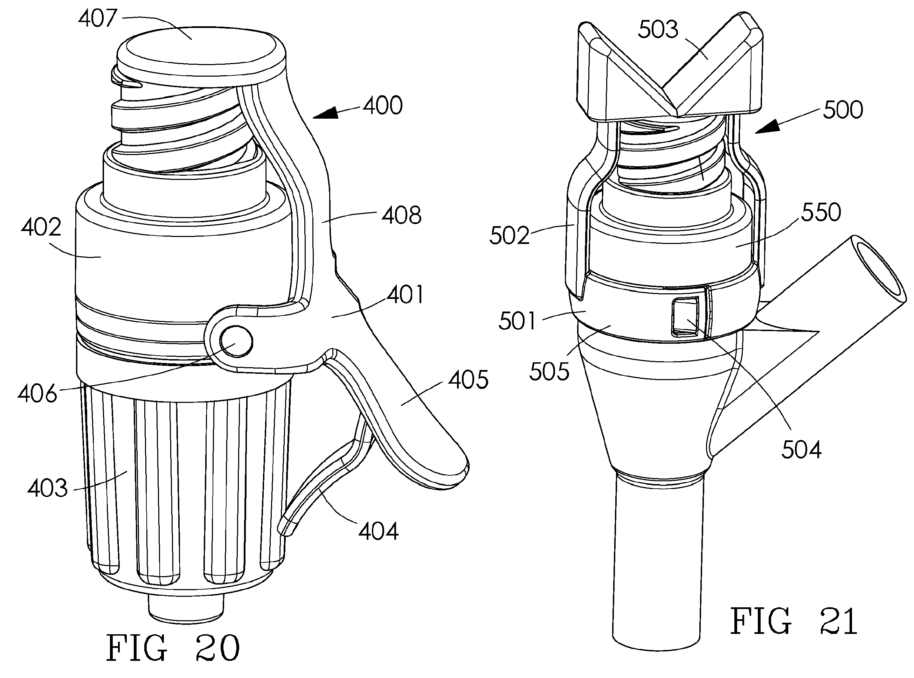

Referring now to FIG. 20, fluid connector cover 400 is shown. Cover body 401 may be attached to the connector housing 402 with a peg and hole assembly 406, which also acts as a rotation or pivot point. Finger-like flexible member 404 connects to lower portion 405 of rigid member 408. Flexible member 404 may provide load to rigid member. Digital pressure to lower portion 405 causes flexible member 404 to deflect against lower connector housing 403 and rigid member 408 to deflect outward, translating cover portion 407 from access site.

Referring now to FIGs. 21-22, automatic fluid connector cover 500 is shown. Cover body 501 may be fastened onto the connector housing 550 by a collar 505 with living hinge 507 and snap-fit element 504. Cover segments 503 connect to flexible arms 502 of the cover body. Insertion of external device 506 spreads cover segments apart to allow access to the access site.

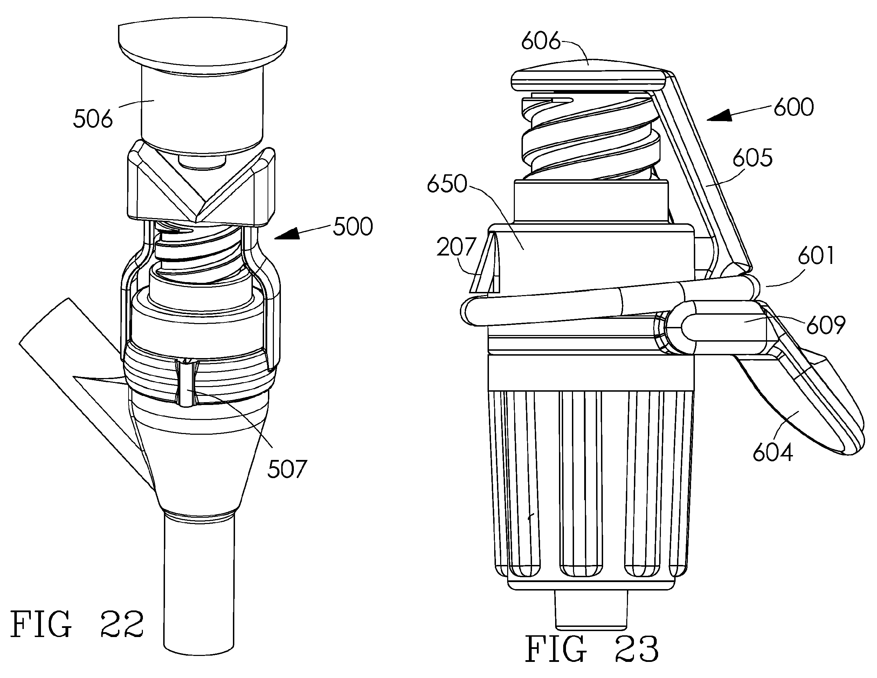

Referring now to FIG. 23, fluid connector cover 600 comprises body member 609 connected to the connector housing 650 between rigid member 605 lower portion 604 and distal end having cover member 606. Elastomeric member 601 holds the cover member 606 adjacent the access site in an initial state. Digital pressure to the lower portion 604 translates cover member 606 from the access site in an are-like motion and provides force to rigid member 605 for returning of the cover member to the access site. Housing retaining element 207 may be integrated with the housing for retaining elastomeric member and/or preventing slippage.

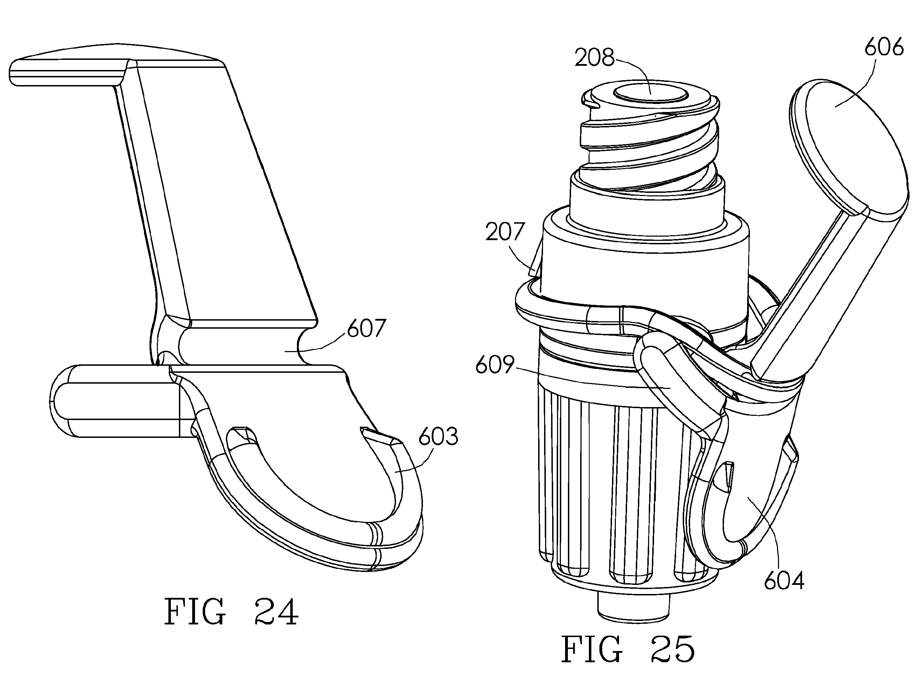

Referring now to FIG. 24, the cover adapter without the connector housing is shown without the elastomeric member. Retaining element for receiving the elastomeric member 607 as well as a grip element 603 for providing finger stability and ergonomic control are depicted.

Referring now to FIG. 25, fluid connector cover 600 is shown in the activated state. Digital pressure to lower portion 604 of the rigid member translates cover member 606 in an arc-like motion, exposing access site 208. The connector housing and/or the retaining element 207 allows the elastomeric member to stretch providing a load for returning the cover member over the access site 208. Elastomeric member may be continuous or multiple elastomeric members that may be individually retained by housing retaining elements provided on the housing.

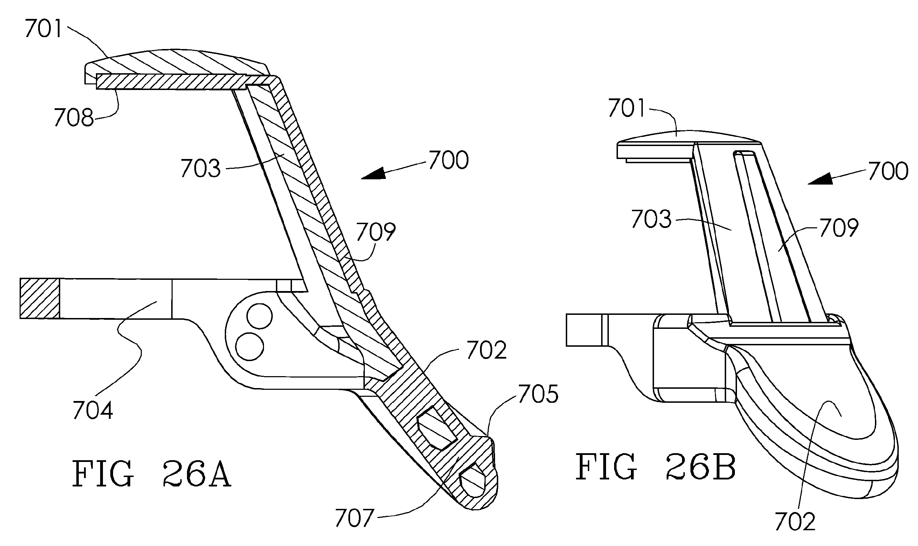

Referring to FIGs. 26A and 26B, cross-sectional and perspective view, respectively, of cover adapter 700 are shown. Cover adapter 700 comprises integrated elastomeric material. The joining method of the cover adapter may include two-shot co-injection, over-molding or adhesion. Rigid member 703 distally terminates to cover portion 701 that may be seated adjacent the access site in the natural state. Rigid member 703 between and/or including the cover member 701 and lower portion 702 may contain elastomeric material, for example by overmolding of the cover adapter. Elastomeric covering surface 708 provides for contact with the access site. Material channel 709 may provide for connecting the elastomeric covering surface with the flexible member 704. The elastomeric material may cover the lower portion 702 as well as provide gripping elements 705. Elements 707 may be provided in the rigid member 703 to assist elastomeric material adhesion to the cover adapter.

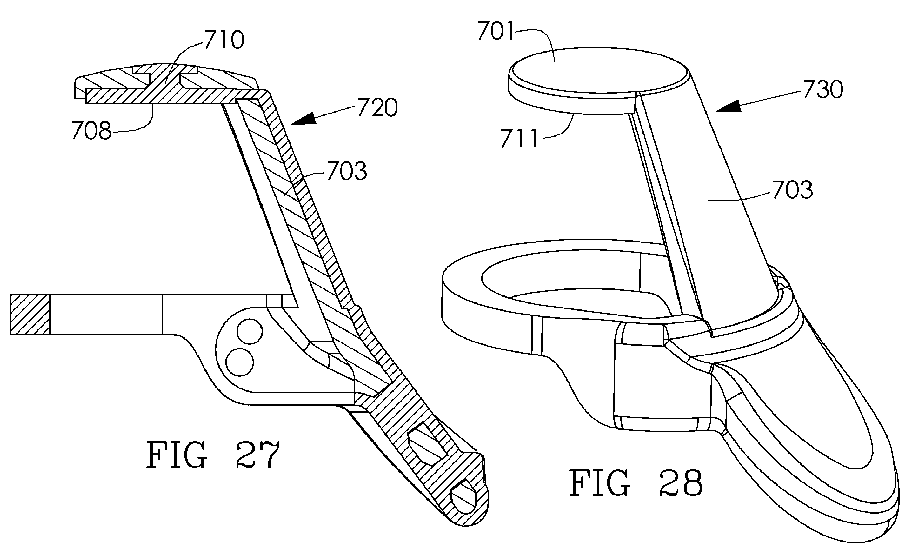

Referring now to FIG. 27, a cross-sectional view of cover adapter 720 is shown. Retaining element 710 provides for securing elastomeric covering surface 708 to the cover member and may provide a gate location for injecting the elastomer material. Cover adapter 730 comprising cover member 711, as an extension of the rigid member 703, without elastomeric material is shown in FIG. 28.

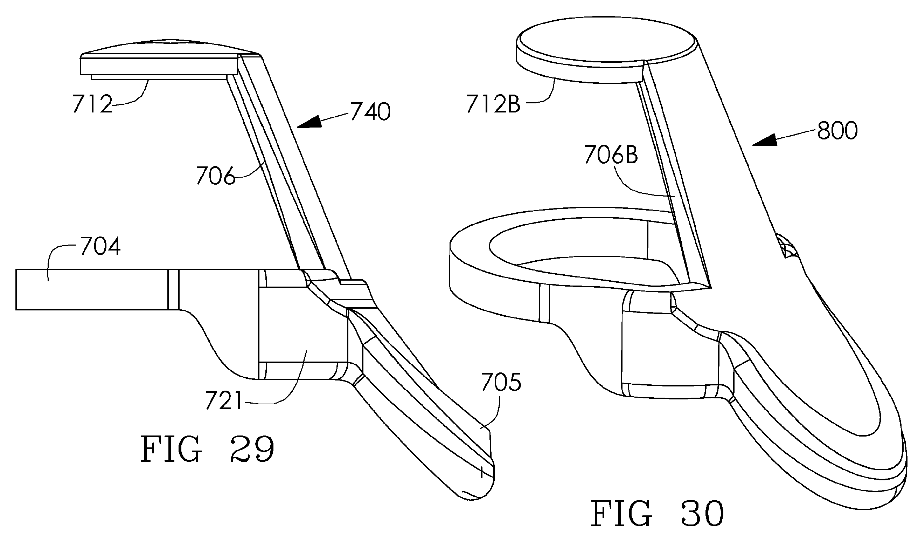

Referring now to FIG. 29, cover adapter 740 is shown. Cover body member 721 comprises rigid member 706 having separate elastomeric bodies; covering surface 712 and flexible member 704. Body member 721 may include gripping elements 705.

Referring now to FIG. 30, cover adapter 800 is shown. Cover member 712B and rigid member 706B may be entirely elastomeric material. Functionality of the adapter may be provided by thin sections for flexibility and thick sections for rigidity.

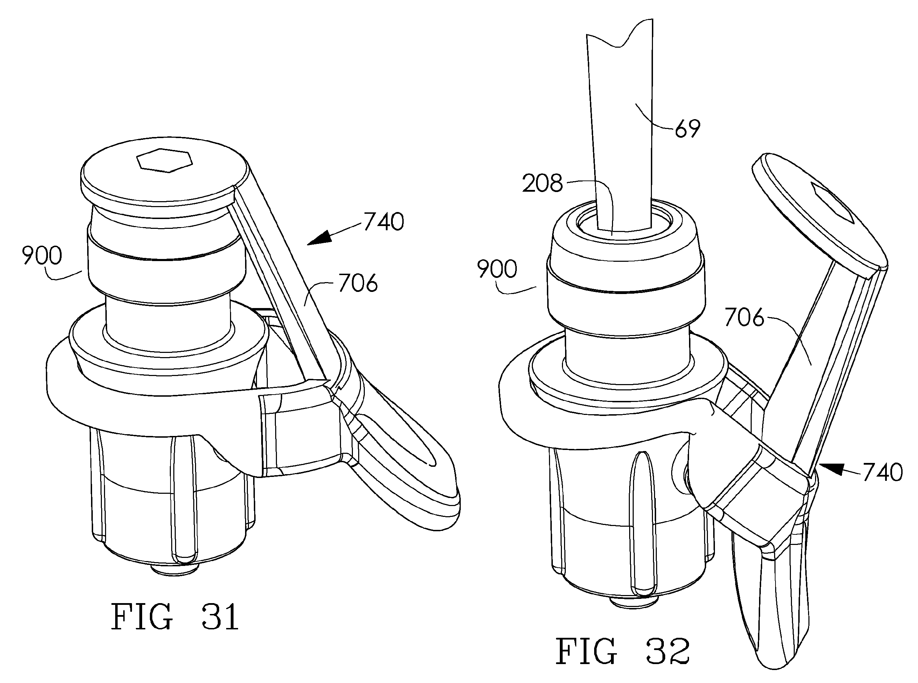

Refering now to FIGs.31 and 32, cover adapter 740 is shown in the initial passive state and an activated state. Penetrable septum type connector 900 is shown with attached cover adapter 740. Penetrating member 69 is provided access to access site 208 when digital force (not shown) is applied to rigid member 706.

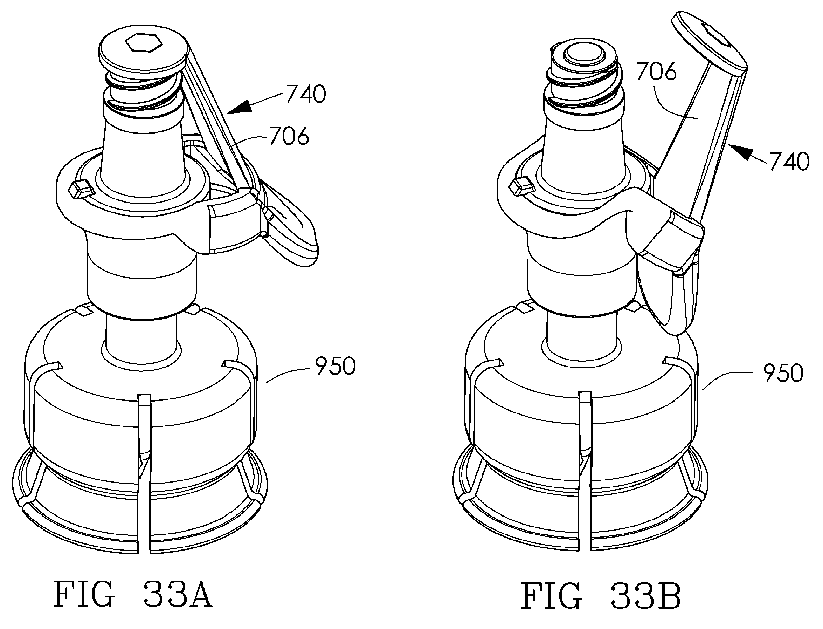

Refering now to FIGs.33A and 33B, cover adapter 740 is shown in the initial passive state and an activated state adapted to vial adapter 950.

The fluid connector cover may be provided in a separate sterile package. The protective cover described in this document is not limited to use with I.V. products, Y-connectors, male/-female luers and the like. Touch contamination is an issue with many connection applications. A protective cover, particularly a passive fluid connector cover as herein described may prevent or eliminate microbial growth and may also minimize particulate and other foreign material from being flushed or otherwise introduced through a fluid connection. Adaptations of the designs could be made to fit the parts to virtually any connection device.

Any and all of the components of the fluid connector cover may be injection molded, compression molded and/or transfer molded. Formed parts may further be overmolded with elastomeric material. Tooling of simple open/close design may be used. Integration of the fluid connector cover with the connector housing may provide a method to incorporate a cover without increasing the number of components, which may reduce assembly cost. Modification of existing female connector housings to provide for fluid connector covers as herein described may utilize tooling of the same orientation as for molding the existing housing. This may minimize cost in modifying current housings for adaptation of the fluid connector cover.

The fluid connector cover described above will normally be supplied in assembled form or as a kit, and may be provided sterile. The term "fluid connector cover kit" as used herein is intended to include within its scope the fluid connector cover or cover adapter thereof in partially or fully disassembled form. The fluid connector cover or kit may contain a fluid connector and a separate fluid connector cover assemblage for user-assembly. The fluid connector cover may be secured to or permanently affixed to the fluid connector.

In this specification and the appended claims, the singular forms "a," "an," and "the" include plural reference unless the context clearly dictates otherwise.

As used herein, "comprising" and grammatical equivalents thereof are inclusive or open-ended terms that do not exclude additional, unrecited elements or method steps. "Comprising" is to be interpreted as including the more restrictive terms "consisting of and "consisting essentially of."

As used herein, "consisting of" and grammatical equivalents thereof exclude any element, step, or ingredient not specified in the claim.

As used herein, "consisting essentially of and grammatical equivalents thereof limit the scope of a claim to the specified materials or steps and those that do not materially affect the basic and novel characteristic or characteristics of the claimed invention.

While the invention has been described in detail and with reference to specific embodiments thereof, it will be apparent to one skilled in the art that various changes and modifications may be made without departing from the scope of the invention.

## Claims

1. A fluid connector comprising:
a housing (202) comprising at least one fluid access site (208) and attachment members (211);
a body member (212) secured to the housing (202) with corresponding attachment members (210, 213);
a rigid arm (204) having a proximal end (205) and a distal end, the rigid arm (204) joined to the body member (212) between the proximal end (205) and the distal end;
a cover member (203) integral with the distal end of the rigid arm (204) and covering the access site (208), the cover member (203) being displaceable from the access site (208) by displacement of the rigid arm (204); and
an elastomeric member (201) loading the rigid arm (204) between the proximal end (205) and the distal end, so that the cover member (203) is under a load such that the cover member (203) is passively held in the covering relationship with the access site (208),
**characterized in that**
the attachment members (211, 210, 213) connecting the body member (212) to the housing (202) provide a pivoting connection.

2. A fluid connector according to claim 1 further comprising housing retaining means (207) to restrict the movement of the elastomeric member (201) to provide load for returning the cover member (203) to its initial state of covering the access site (208).

3. A fluid connector according to any preceding claim, wherein any surface of the connector may include, or be impregnated with, an aseptically effective material, preferably wherein the aseptically effective material comprises an anti-microbial agent.

4. A fluid connector according to any preceding claim, wherein the cover member (203) comprises an aseptically effective agent.

5. A fluid connector according to any preceding claim, wherein the cover member (203) contains an antimicrobial agent.

6. A fluid connector according to any preceding claim, wherein the cover member (203) comprises a separate element containing an anti-microbial agent, preferably wherein the separate element comprises sponge, foam or absorbable material.

7. A fluid connector according to any of claims 4 to 6, wherein the anti-microbial agent is selected from antibacterial agent, antiviral agent, antifungal agent, anti-parasitic drug.

8. A fluid connector according to any preceding claim, wherein the access site (208) comprises a material capable of providing entry by a piercing, penetrating, or inserting device.

9. A fluid connector according to any preceding claim, wherein the cover member (203) is in direct contact with the access site (208).

10. A fluid connector according to any preceding claim, wherein the cover member (203) provides an airtight seal with the fluid access site (208).

11. A fluid connector according to any preceding claim, wherein the housing (202) is formed of a thermoplastics material.

12. A fluid connector according to any preceding claim, further comprising a shroud element, preferably wherein the shroud element at least partially surrounds the cover member (203) such as to cover the sides of the access site (208).

13. A fluid connector according to any preceding claim, wherein the cover member (203) comprises one or more cutouts configured to allow the cover member (203) to flex, when the cover member (203) at least partially mates with one or more engaging elements on the housing (202).

14. A fluid connector according to any preceding claim, wherein the cover member (203) comprises an elastomeric seal.

## Patentansprüche

1. Fluidverbinder mit:
einem Gehäuse (202) mit mindestens einem Fluidzugangsort (208) und einem Anbringelement (211);
einem Körperelement (212), das an dem Gehäuse (202) mit entsprechenden Anbringelementen (210, 213) gesichert ist;
einem starren Arm (204), der ein nahes Ende (205) und ein fernes Ende hat, wobei der starre Arm (204) an dem Körperelement (212) zwischen dem nahen Ende (205) und dem fernen Ende angeschlossen ist;
einem Abdeckelement (203), das mit dem fernen Ende des starren Arms (204) aus einem Stück ist und den Zugangsort (208) abdeckt, wobei das Abdeckelement (203) von dem Zugangsort (208) durch Verlagerung des starren Arms (204) verlagerbar ist; und
einem elastomeren Element (201), das den starren Arm (204) zwischen dem nahen Ende (205) und dem fernen Ende spannt, so dass das Abdeckelement (203) unter einer Last steht, so dass das Abdeckelement (203) passiv in der Abdeckbeziehung mit dem Zugangsort (208) gehalten ist,
**dadurch gekennzeichnet, dass**
die Anbringelemente (211, 210, 213), die das Körperelement (212) mit dem Gehäuse (202) verbinden, eine Schwenkverbindung bereitstellen.

2. Fluidverbinder nach Anspruch 1, ferner mit Gehäusesicherungsmitteln (207), um die Bewegung des elastomeren Elements (201) zu beschränken, um eine Last zum Rückführen des Abdecklements (203) zu seinem Anfangszustand des Abdeckens des Zugangsorts (208) bereitzustellen.

3. Fluidverbinder nach einem der vorhergehenden Ansprüche, wobei eine Fläche des Verbinders ein aseptisch wirkendes Material umfassen oder mit diesem impregniert sein kann, vorzugsweise wobei das aseptisch wirkende Material ein antimikrobielles Mittel ausweist.

4. Fluidverbinder nach einem der vorhergehenden Ansprüche, wobei das Abdeckelement (203) ein aseptisch wirkendes Mittel aufweist.

5. Fluidverbinder nach einem der vorhergehenden Ansprüche, wobei das Abdeckelement (203) ein antimikrobielles Mittel enthält.

6. Fluidverbinder nach einem der vorhergehenden Ansrpüche, wobei das Abdeckelement (203) ein separates Teil aufweist, das ein antimikrobielles Mittel enthält, vorzugsweise wobei das separate Teil einen Schwamm, Schaumstoff oder ein absorptionsfähiges Material aufweist.

7. Fluidverbinder nach irgendeinem der Ansrpüche 4 bis 6, wobei das antimikrobielle Mittel von einem antibakteriellen Mittel, einem antiviralen Mittel, einem antimykotischen Mittel oder einem antiparasitischen Arzneimittel ausgewählt ist.

8. Fluidverbinder nach einem der vorhergehenden Ansrpüche, wobei der Zugangsort (208) ein Material aufweist, das in der Lage ist, einen Zugang mittels einer Durchstechungs-, Durchdringungs- oder Einführungsvorrichtung bereitzustellen.

9. Fluidverbinder nach einem der vorhergehenden Ansrpüche, wobei das Abdeckelement (203) in direktem Kontakt mit dem Zugangsort (208) ist.

10. Fluidverbinder nach einem der vorhergehenden Ansrpüche, wobei das Abdeckelement (203) eine luftdichte Dichtung mit dem Fluidzugangsort (208) bereitstellt.

11. Fluidverbinder nach einem der vorhergehenden Ansrpüche, wobei das Gehäuse (202) aus einem thermoplastischen Material ausgebildet ist.

12. Fluidverbinder nach einem der vorhergehenden Ansrpüche, ferner mit einem Kragenteil, vorzugsweise wobei das Kragenteil zumindest teilweise das Abdeckelement (203) umgibt, so dass es die Seiten des Zugangsorts (208) abdeckt.

13. Fluidverbinder nach einem der vorhergehenden Ansrpüche, wobei das Abdeckelement (203) einen oder mehrere Ausschnitte aufweist, die eingerichtet sind, um dem Abdeckelement (203) zu erlauben, sich zu beugen, wenn das Abdeckelement (203) zumindest teilweise mit einem oder mehreren Eingriffelementen an dem Gehäuse (202) in Eingriff tritt.

14. Fluidverbinder nach einem der vorhergehenden Ansrpüche, wobei das Abdeckelement (203) eine elastomere Dichtung aufweist.

## Revendications

1. Connecteur de fluide comprenant :
un logement (202) comprenant au moins un site (208) d'accès de fluide et des éléments (211) de fixation ;
un élément (212) de corps fixé au logement (202) avec des éléments (210, 213) de fixation correspondants ;
un bras rigide (204) ayant une extrémité proximale (205) et une extrémité distale, le bras rigide (204) joint à l'élément (212) de corps entre l'extrémité proximale (205) et l'extrémité distale ;
un élément (203) de couvercle intégré avec l'extrémité distale du bras rigide (204) et recouvrant le site (208) d'accès, l'élément (203) de couvercle étant déplaçable depuis le site (208) d'accès par déplacement du bras rigide (204) ; et
un élément élastomérique (201) chargeant le bras rigide (204) entre l'extrémité proximale (205) et l'extrémité distale, de telle sorte que l'élément (203) de couvercle est sous une charge telle que l'élément (203) de couvercle est maintenu passivement dans la relation de recouvrement avec le site (208) d'accès,
**caractérisé en ce que**
les éléments (211, 210, 213) de fixation connectant l'élément (212) de corps au logement (202) offrent une connexion pivotante.

2. Connecteur de fluide selon la revendication 1, comprenant en outre un moyen (207) de maintien de logement pour limiter le mouvement de l'élément élastomérique (201) pour fournir une charge pour remettre l'élément (203) de couvercle en son état initial de recouvrement du site (208) d'accès.

3. Connecteur de fluide selon une quelconque revendication précédente, dans lequel une quelconque surface du connecteur peut inclure, ou être imprégnée avec, une matière aseptiquement efficace, préférablement dans lequel la matière aseptiquement efficace comprend un agent antimicrobien.

4. Connecteur de fluide selon une quelconque revendication précédente, dans lequel l'élément (203) de couvercle comprend un agent aseptiquement efficace.

5. Connecteur de fluide selon une quelconque revendication précédente, dans lequel l'élément (203) de couvercle comprend un agent antimicrobien.

6. Connecteur de fluide selon une quelconque revendication précédente, dans lequel l'élément (203) de couvercle comprend un élément séparé contenant un agent antimicrobien, préférablement dans lequel l'élément séparé comprend une éponge, une mousse ou une matière absorbable.

7. Connecteur de fluide selon l'une quelconque des revendications 4 à 6, dans lequel l'agent antimicrobien est choisi parmi un agent antibactérien, un agent antiviral, un agent antifongique, un médicament antiparasitaire.

8. Connecteur de fluide selon une quelconque revendication précédente, dans lequel le site (208) d'accès comprend une matière apte à permettre l'entrée par un dispositif de perçage, de pénétration, ou d'insertion.

9. Connecteur de fluide selon une quelconque revendication précédente, dans lequel l'élément (203) de couvercle est en contact direct avec le site (208) d'accès.

10. Connecteur de fluide selon une quelconque revendication précédente, dans lequel l'élément (203) de couvercle offre un joint étanche à l'air avec le site (208) d'accès de fluide.

11. Connecteur de fluide selon une quelconque revendication précédente, dans lequel le logement (202) est constitué d'un matériau thermoplastique.

12. Connecteur de fluide selon une quelconque revendication précédente, comprenant en outre un élément d'enveloppe de protection, préférablement dans lequel l'élément d'enveloppe de protection entoure au moins partiellement l'élément (203) de couvercle de façon à recouvrir les côtés du site (208) d'accès.

13. Connecteur de fluide selon une quelconque revendication précédente, dans lequel l'élément (203) de couvercle comprend une ou plusieurs découpe(s) configurée(s) pour permettre que l'élément (203) de couvercle fléchisse, lorsque l'élément (203) de couvercle s'ajuste au moins partiellement avec un ou plusieurs élément(s) d'engagement sur le logement (202).

14. Connecteur de fluide selon une quelconque revendication précédente, dans lequel l'élément (203) de couvercle comprend un joint élastomérique.
